# EUROPEAN PATENT APPLICATION

(11) **EP 2 583 694 A1**
(43) Date of publication of application: **24.04.2013**
(21) Application number: 11795859.5
(22) Date of filing: 15.06.2011
(51) Int. Cl.: A61L 2/18, B01F 1/00, B01F 3/04, B01F 5/00, B01F 5/12, B08B 3/02, B08B 3/08, C02F 1/28, C02F 1/30, C02F 1/50, C02F 1/72, C02F 1/78

(54) **WASHING/STERILISATION DEVICE**

(30) Priority: 18.06.2010 JP 2010138981
(71) Applicant: ACP Japan Co. Ltd., Tokyo 113-0033 (JP); Nakamura, Shoichi, Higashichikuma-gun Nagano 399-7502 (JP)
(72) Inventor: NAKAMURA, Shoichi, Nagano 399-7502 (JP)
(74) Representative: Gassner, Wolfgang
(86) International application number: PCT/JP2011/064188
(87) International publication number: WO 2011/158957

(57) **Abstract**

To provide a cleaning sterilization apparatus capable of sterilizing an object to be cleaned such as a medical instrument effectively in a short time by generating and using ozone water containing ozone with particle diameters hard to disappear in water, the cleaning sterilization apparatus is provided with cleaning water supply means, ozone water generating means, and squirting means for squirting cleaning water and ozone water toward the obj ect to be cleaned inside a container, where the ozone water generating means is provided with a mixing pump which takes in ozone and water to mix and generates ozone-mixed water, ozone supply means for supplying ozone to the mixing pump, stirring means for colliding the ozone-mixed water from the mixing pump sequentially with a plurality of protrusions with running-water pressure applied inside an enclosed running-water channel with the plurality of protrusions arranged therein, and thereby making ozone contained in the ozone-mixed water finer to generate ozone water, and a line mixer which causes the stirring means to circulate and stir the ozone-mixed water generated by the mixing pump and thereby adjusts particle diameters of ozone contained in the ozone-mixed water.

## Description

### Technical Field

The present invention relates to a cleaning sterilization apparatus for cleaning and sterilizing an object to be cleaned such as a medical instrument using ozone water, and more particularly, to a cleaning sterilization apparatus with decomposition/oxidation action improved for poisonous organic substances such as viruses and bacteria by using ozone water with particle diameters of contained ozone bubbles optimized.

### Background Art

Medical instruments such as a surgical knife, scissors and clamp used in the medical field need sufficient cleaning and sterilization after being used to prevent secondary infection of a virus and bacteria contained in body fluid such as blood and lymph. For cleaning and sterilization treatment of medical instruments, to prevent infection to a cleaning operator that handles the instruments, it is necessary to use a cleaning sterilization apparatus for automatically performing the cleaning treatment and sterilization treatment in series while avoiding operation by human as possible.

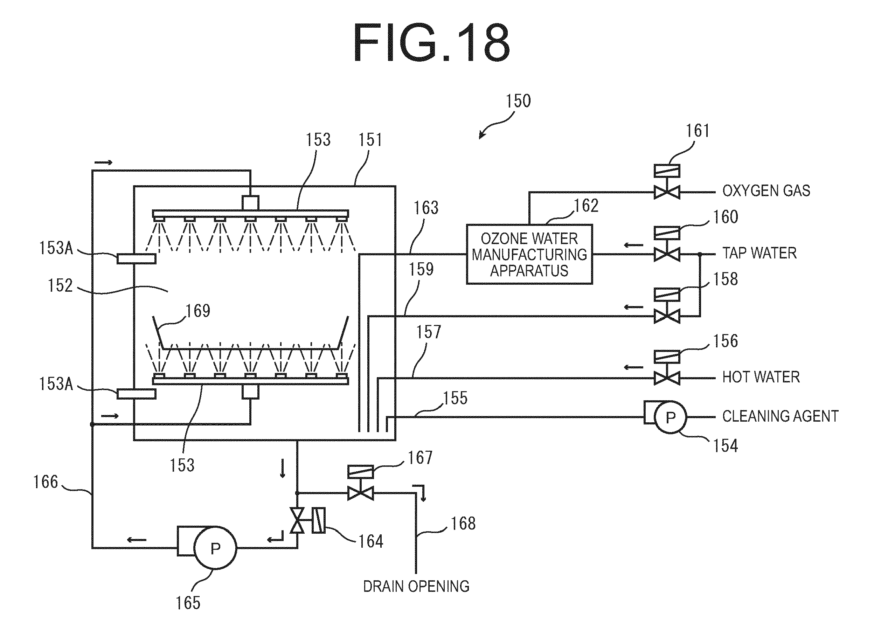

FIG. 18 is a schematic configuration diagram showing an example of a common cleaning sterilization apparatus used in the medical field. A cleaning sterilization apparatus 150 as shown in FIG. 18 is provided with a housing 151 with an openable/closable cover formed on its top, two rotating nozzles 153 respectively disposed on the upper side and lower side of a cleaning and sterilization space 152 formed inside the housing 151, two water level sensors 153A respectively disposed on the upper side and lower side inside the cleaning and sterilization space 152, a cleaning agent pump 154 which takes in a cleaning agent from a cleaning agent tank and supplies the cleaning agent into the cleaning and sterilization space 152 via a pipe 155, a solenoid valve 156 which takes in hot water from a hot water storage tank to supply to the cleaning and sterilization space 152 via pipe 157, a solenoid valve 158 which takes in tap water or the like to supply to the cleaning and sterilization space 152 via a pipe 159, and a solenoid valve 160 which takes in tap water or the like.

Further, the apparatus is provided with a solenoid valve 161 which takes in an oxygen gas, an ozone water manufacturing apparatus 162 which generates ozone water using the tap water and oxygen gas respectively supplied from the solenoid valves 160, 161 and supplies the ozone water to the cleaning and sterilization space 152 via a pipe 163, a solenoid valve 164 which takes in the cleaning water and the like accumulated on the bottom inside the cleaning and sterilization space 152, a circulating pump 165 which takes in the cleaning water and the like supplied via the solenoid valve 164 to supply to each of the rotating nozzles 153 via a pipe 166, and a solenoid valve 167 which takes in the cleaning water, ozone water and the like accumulated on the bottom inside the cleaning and sterilization space 152 to discharge to the outside via a pipe 168.

In using the conventional cleaning sterilization apparatus 150, first, a user opens the cover, sets a basket 169 with used medical instruments therein on the cleaning and sterilizing space 152, and closes the cover. Next, when a start button is pressed, each of the solenoid valves 154, 156 and the like is operated, hot water (cleaning water) containing a cleaning agent is generated and is stored inside the cleaning and sterilization space 152.

Subsequently, each of the solenoid valves 154, 156 and the like is returned to the non-operating state, while the solenoid vale 164 and circulating pump 165 are operated, and the cleaning water inside the cleaning and sterilization space 152 is introduced to each of the rotating nozzles 153, and is squirted to the medical instruments to clean the instruments.

After a lapse of certain time in this state, the solenoid valve 164 and circulating pump 165 are returned to non-operating states, cleaning of the medical instruments is finished, and then, the cleaning water inside the cleaning and sterilization space 152 is drained to the outside by controlling the solenoid valve 167 and the like.

Next, after finishing the drain processing of the cleaning water, the solenoid valve 167 is returned to the non-operating state, while each of the solenoid valves 160, 161 and the ozone water manufacturing apparatus 162 are operated for a certain time, a certain amount of ozone water is discharged from the ozone water manufacturing apparatus 162, and the ozone sterilization treatment is performed on the medical instruments for a certain time with the ozone water stored inside the cleaning and sterilization space 152.

When the certain time has elapsed and the ozone sterilization treatment of the medical instruments is completed, the solenoid valve 167 and the like are operated, the ozone water inside the cleaning and sterilization space 152 is drained to the outside, and the cleaning and sterilization treatment of the medical instruments is completed.

### Disclosure of the Invention

### Problems to be Solved by the Invention

In addition, in such a conventional cleaning sterilization apparatus 150, a large amount of ozone is injected into tap water, and high-concentration ozone water is generated to obtain a high degree of sterilizing effect.

At this point, when the particle diameter of an ozone bubble contained in the ozone water is increased, ozone floats in the ozone water and bursts in the water surface, and contact characteristics between ozone and the object to be cleaned extremely degrade. Therefore, the ozone water manufacturing apparatus 162 is configured to generate ozone water containing ozone with sufficiently small particle diameters in the range of 0.5 µm to 3 µm.

However, ozone with such small particle diameters gradually further decreases in the diameter inside the ozone water, and finally is crushed and disappears, and therefore, it is difficult to increase the concentration of ozone in ozone water.

As a result, effective destruction is not performed on organic tissue such as organic substances, viruses and bacteria adhering to the medical instruments, and there is the problem that it is necessary to not only increase the usage amount of ozone water corresponding thereto, but also increase the ozone treatment time.

The present invention was made in view of the aforementioned circumstances, and it is an object of the invention to provide a cleaning sterilization apparatus capable of sterilizing an object to be cleaned effectively in a short time by generating and using ozone water containing ozone with particle diameters hard to disappear in water.

### Means for Solving the Problem

To attain the aforementioned object, the present invention provides a cleaning sterilization apparatus which cleans an object to be cleaned by squirting cleaning water with the object to be cleaned held inside a container, while sterilizing and disinfecting the object to be cleaned by oxidation-decomposing organic substances such as a virus, bacteria or the like adhering to the object to be cleaned using ozone water, and is characterized by having cleaning water supply means for supplying cleaning water, ozone water generating means for generating ozone water to supply, and squirting means for squirting the cleaning water and the ozone water toward the object to be cleaned inside the container, where the ozone water generating means is provided with a mixing pump which takes in ozone and water to mix, and generates ozone-mixed water with ozone mixed into water, ozone supply means for supplying ozone to the mixing pump, stirring means for colliding the ozone-mixed water supplied from the mixing pump sequentially with a plurality of protrusions with running-water pressure applied inside an enclosed running-water channel with the plurality of protrusions arranged therein, and thereby making ozone contained in the ozone-mixed water finer to generate ozone water, and a line mixer which causes the stirring means to circulate and stir the ozone-mixed water generated by the mixing pump and thereby adjusts particle diameters of ozone contained in the ozone-mixed water.

In addition, in the description, water with ozone simply mixed thereinto is called ozone-mixed water, and ozone-mixed water with particle diameters of contained ozone bubbles optimized, which is eventually generated by the ozone water generating means of the invention, is referred to as "ozone water".

Herein, the cleaning sterilization apparatus preferably squirts the ozone water to the object to be cleaned by the squirting means while circulating the ozone water, and thereby sterilizes and disinfects the object to be cleaned inside the container. Alternately, the apparatus may circulate the ozone water at a predetermined flow rate with the object to be cleaned immersed in the ozone water and thereby sterilize and disinfect the object to be cleaned inside the container.

In addition, the apparatus may be further provided with ultrasonic vibration means for vibrating the ozone water at a frequency of an ultrasonic band inside the container.

Herein, it is suitable that the stirring means has a circular stirring plate with a plurality of protrusions spaced circumferentially formed therein, collides the ozone-mixed water sequentially with the plurality of protrusions, and thereby makes ozone contained in the ozone-mixed water finer. Alternately, the stirring means may have a stirring block in the shape of a cylinder with a plurality of protrusions spaced circumferentially formed in a tapered inner surface of the cylinder, collide the ozone-mixed water sequentially with the plurality of protrusions, and thereby make ozone contained in the ozone water finer.

Then, it is suitable that particle diameters of ozone bubbles inside the ozone water are controlled to within a desired size range by adjusting the running-water pressure inside the stirring means by the mixing pump and time of circulation stirring by the line mixer.

Further, a discharge pressure of the ozone-mixed water by the mixing pump which is supplied to the stirring means by the mixing pump preferably ranges from 3 to 8 atmospheres. Furthermore, it is suitable that the desired size range of particle diameters of the ozone bubbles ranges from 4 to 50 µm.

Moreover, it is suitable that the mixing pump takes in the cleaning water and mixes the cleaning water with the ozone water and that the squirting means squirts the cleaning water and the ozone water toward the object to be cleaned.

Herein, the cleaning water may contain a hydrogen peroxide solution as well as the cleaning agent. Further, as well as the cleaning agent, the cleaning water may contain a photocatalyst, apatite or photocatalyst-apatite alone or a complex thereof.

In addition, it is suitable that an ozone supply apparatus generates an ozone gas with an ozone concentration ranging from 70 to 120 g/m3 to supply to the mixing pump.

### Advantageous Effect of the Invention

According to cleaning and sterilization of the invention, by optimizing particle diameters of ozone and thereby using ozone water containing ozone bubbles hard to disappear in water, it is possible to improve decomposition/oxidation action on poisonous organic substances such as viruses and bacteria. Therefore, it is possible to sterilize an object to be cleaned such as a medical instrument in a short time.

### Brief Description of Drawings

FIG. 1 is a schematic configuration diagram showing an Embodiment of a cleaning sterilization apparatus according to the invention;
FIG. 2 is a functional block diagram showing a specific functional configuration example of the cleaning sterilization apparatus as shown in FIG. 1;
FIG. 3 is a flowchart showing an operation example of the cleaning sterilization apparatus as shown in FIG. 1;
FIG. 4 is another flowchart showing the operation example of the cleaning sterilization apparatus as shown in FIG. 1;
FIG. 5 is a perspective view showing an example of an ozone water generating apparatus used in the cleaning sterilization apparatus according to the invention;
FIG. 6 is a partly-cut plan view of the ozone water generating apparatus as shown in FIG. 5;
FIG. 7 is a partly-cut front view of the ozone water generating apparatus as shown in FIG. 5;
FIG. 8 is a sectional view of a line mixer as shown in FIG. 7;
FIG. 9 is sectional view of a stirring portion as shown in FIG. 8;
FIG. 10 contains diagrams showing the frontside and backside of a stirring plate as shown in FIG. 9;
FIG. 11 is a graph showing the relationship between the size and duration of ozone contained in ozone water generated by the ozone water generating apparatus as shown in FIG 5;
FIG. 12 is a front view showing another example (I) of stirring means used in the invention;
FIG. 13 is a line A-A sectional view taken along the line A-A of a stirring block as shown in FIG. 12;

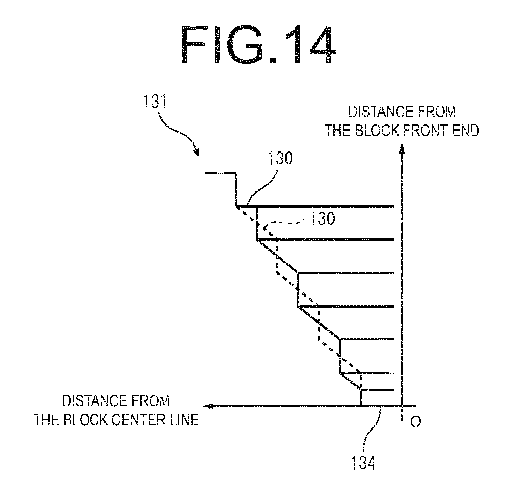

FIG. 14 is a diagram showing the relationship in each stirring surface as shown in FIG. 12;
FIG. 15 is a front view showing still another example (II) of stirring means used in the invention;
FIG. 16 is a line B-B sectional view taken along the line B-B of a stirring block as shown in FIG. 15;
FIG. 17 is a diagram showing the relationship in each stirring surface as shown in FIG. 15; and
FIG. 18 is a schematic configuration diagram showing an example of a common cleaning sterilization apparatus used in the medical field.

### Best Mode for Carrying Out the Invention

### «Description of ozone water generating apparatus»

First, prior to the specific description of a cleaning sterilization apparatus according to the invention, described is an ozone water generating apparatus used in the cleaning sterilization apparatus of the invention.

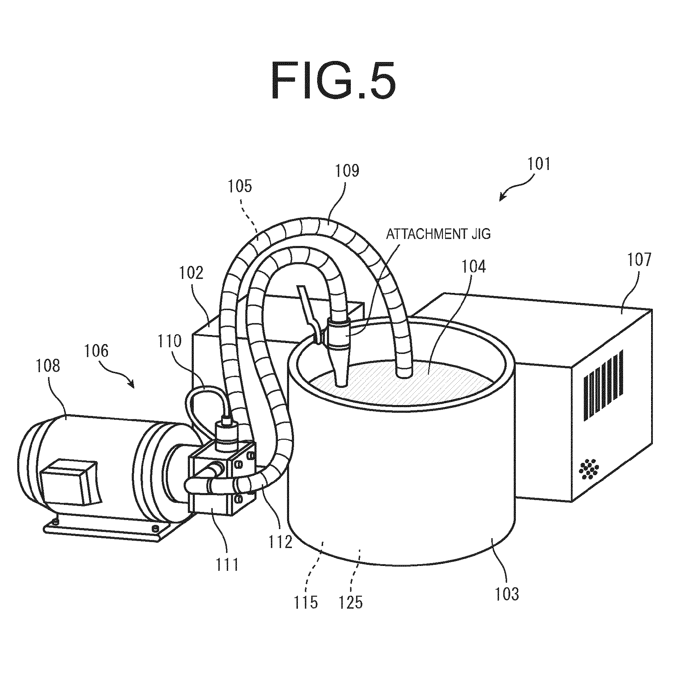

FIG. 5 is a schematic diagram showing an example of a configuration of the ozone water generating apparatus used in the cleaning sterilization apparatus of the invention.

As shown in the figure, the ozone water generating apparatus 101 is provided with an ozone water supply apparatus 102 that generates ozone, and an ozone water generation dispersion apparatus 106 having a mixing pump 111 and line mixer 115. Further, the ozone water generating apparatus 101 is provided with a power supply apparatus 107 that supplies a power-supply voltage to the ozone water generation dispersion apparatus 106.

In addition, as an object to be cleaned and sterilized in the cleaning sterilization apparatus of the invention, a used medical instrument 16 is mainly targeted, but other than the medical instrument, it is possible to use the cleaning sterilization apparatus in cleaning and sterilization of various articles. For example, it is possible to use the apparatus for barber/hair salon instruments, hygiene items, care items, cooking devices, etc.

In the ozone water generating apparatus 101, the ozone supply apparatus 102 generates ozone to supply to the ozone water generation dispersion apparatus 106, and the ozone water generation dispersion apparatus 106 takes in treatment-target water 104 from a water tank (container) 103, mixes the treatment-target water 104 and ozone, thereby generates ozone water 125 containing ozone made micro bubbles (with particle diameters ranging from 4 to 50 µm), and injects and disperses the ozone water 125 into the treatment-target water 104 inside the water tank 103. The ozone made micro bubbles with particle diameters ranging from 4 to 50 µm has long duration in discharged water, and exerts the greatest action effect of ozone such as sterilization and disinfection. Therefore, in the ozone water generating apparatus 101, the ozone water 125 containing ozone bubbles with the aforementioned particle diameters is generated and injected into the treatment-target water 104, and it is thereby possible to oxidation-decompose organic substances and the like in the treatment-target water 104 efficiently in a short time.

Each component of the ozone water generating apparatus 101 will be described below.

First, the ozone supply apparatus 102 is provided with a high-voltage generator that generates a high voltage, a plurality of discharge tubes for performing corona discharge using the high voltage output from the high-voltage generator, etc. introduces oxygen supplied from an oxygen source into each discharge tube, generates an ozone gas with the ozone concentration ranging from 70 to 120 g/m3 by discharge processing, and supplies an amount of the ozone gas corresponding to a required amount of ozone water to the mixing pump 111. As the oxygen source, used are an oxygen generating apparatus which absorbs surrounding air, adsorbs and removes nitrogen in the air with synthetic zeolite or the like, and thereby generates oxygen, an oxygen cylinder, etc. The generated ozone is supplied to the ozone water generation dispersion apparatus 106 from the pipe 110.

The power-supply apparatus 107 is provided with an AC-DC conversion circuit that rectifies a utility voltage supplied from utility power with a plurality of commutators or the like to generate a direct-current voltage, and an inverter circuit which chops the direct-current voltage obtained by the AC-DC conversion circuit with a plurality of thyristors or the like, and generates a power-supply voltage with designated voltage value and current value, uses the utility voltage supplied from utility power, and generates the power-supply voltage with predetermined high voltage and frequency to supply to the ozone water generation dispersion apparatus 106. In addition, in remote areas or the like to which utility power is not supplied, it is preferable to use power obtained by a solar panel system, wind power system or the like.

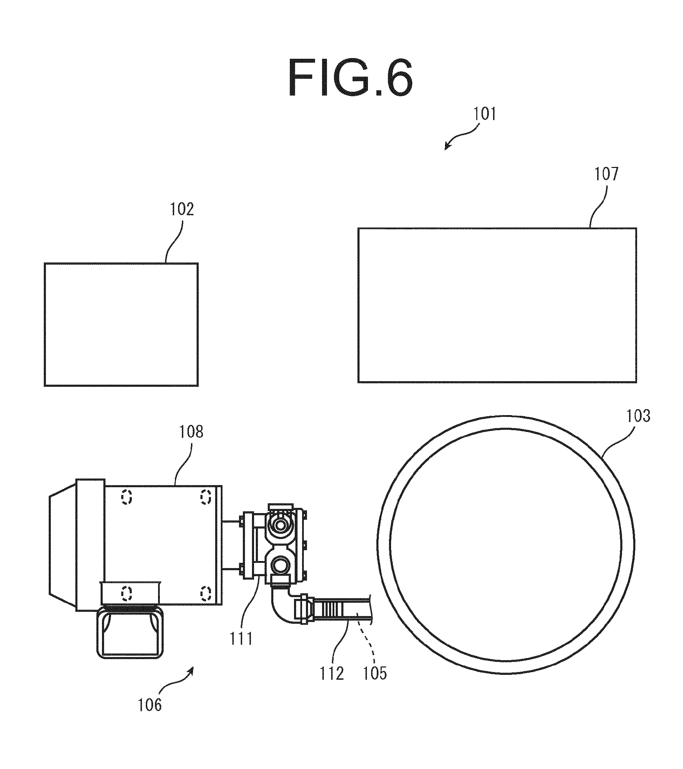

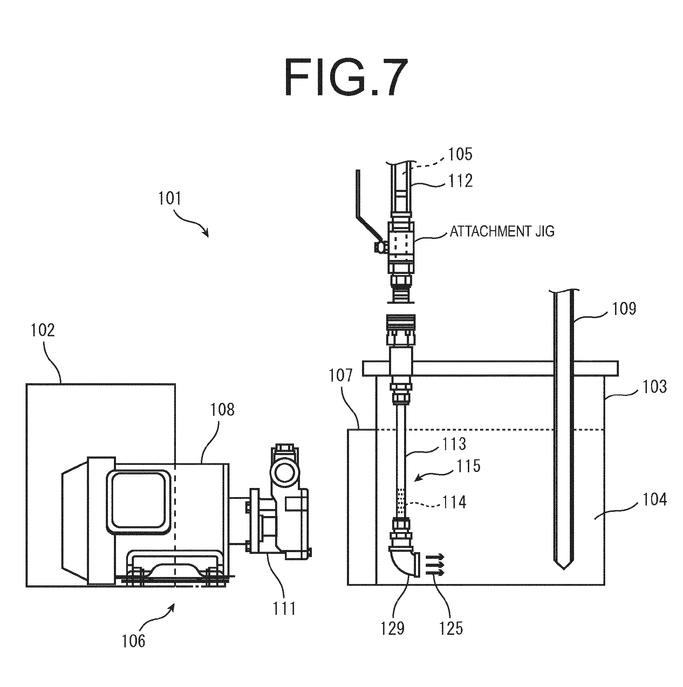

Next, as shown in FIGs. 6 and 7, the ozone water generation dispersion apparatus 106 is provided with a motor 108, mixing pump 111, and line mixer 115. When the power-supply voltage is supplied from the power-supply apparatus 107, the ozone water generation dispersion apparatus 106 absorbs the treatment-target water 104 stored in the water tank 103, and mixes the water with ozone generated in the ozone supply apparatus 102 to generate ozone-mixed water 105. Next, the apparatus 106 stirs the ozone-mixed water 105 with the line mixer, thereby makes ozone water 125 containing micro-bubble ozone that is hard to disappear in the treatment-target water 104, and injects and disperses the ozone water 125 into the treatment-target water in the water tank 103.

Herein, when the power-supply voltage is supplied from the power-supply apparatus 107, the motor 108 rotates the driving shaft at the number of revolutions according to the voltage value and frequency of the power-supply voltage. The mixing pump 111 is fixed to the front end side of the motor 108. When the driving shaft of the motor 108 is driven and rotated, the mixing pump 111 absorbs the treatment-target water 104 inside the water tank 103 via a flexible pipe 109 by operation of impellers connected to the driving shaft, while mixing ozone from the ozone supply apparatus 102 and the treatment-target water 104, and generates the ozone-mixed water 105. The generated ozone-mixed water 105 is discharged at a designated discharge pressure (for example, in the range of 3.5 to 8 atmospheres) and discharge flow rate. A flexible pipe 112 is connected to a discharge opening of the mixing pump 111, and the discharged ozone-mixed water 105 is guided to the line mixer 115 from the flexible pipe 112.

As shown in FIG. 7, the line mixer 115 is comprised of a hollow pipe 113 connected to the front end of the flexible pipe 112, and a plurality of stirring portions (stirring means) 114 disposed on the inner surface of the hollow pipe 113 in intimate contact therewith. The line mixer 115 stirs the ozone-mixed water 105 supplied to the hollow pipe 113 via the flexible pipe 112, for each of the stirring portions 114, and makes ozone inside the ozone-mixed water 105 micro bubbles to generate the ozone water 125. In addition, on the front end side of the line mixer 115 is provided a nozzle 129 for injecting and dispersing the ozone water 125 generated by the line mixer 115 into the treatment-target water 104 inside the water tank 103.

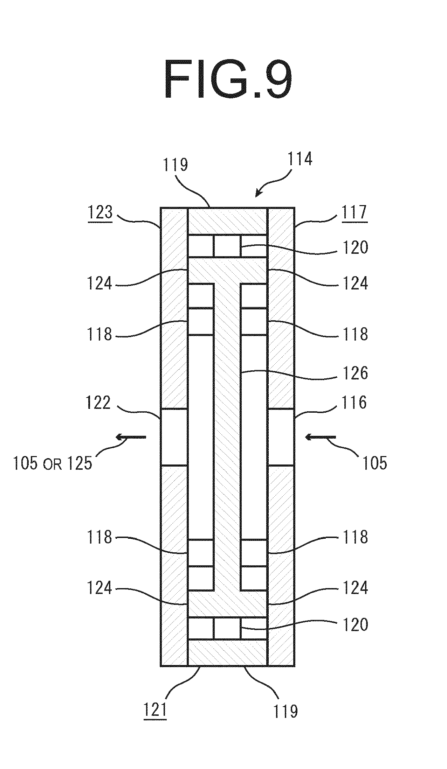

FIG. 9 is a diagram showing the section of the stirring portion 114. Each stirring portion 114 is comprised of an incurrent plate 117 made of a disk member with a diameter (for example, 35 mm) brought into intimate contact with the inner surface of the hollow pipe 113, stirring plate 121 and excurrent plate 123.

The incurrent plate 117 is a disk member having a circular hole 116 in the center, and passes the ozone-mixed water 105 supplied via the flexible pipe 112 through the circular hole 116. The stirring plate 121 is comprised of a disk member 126 disposed so that the frontside is brought into intimate contact with the incurrent plate 117 and that backside is brought into intimate contact with the excurrent plate 123, and stirs the ozone-mixed water 105 supplied via the incurrent plate 117 to make ozone finer. The excurrent plate 123 is a disk member having a circular hole 122 in the center, and discharges the ozone-mixed water 105 gathered in the center portion of the backside of the stirring plate 121 from the circular hole 122.

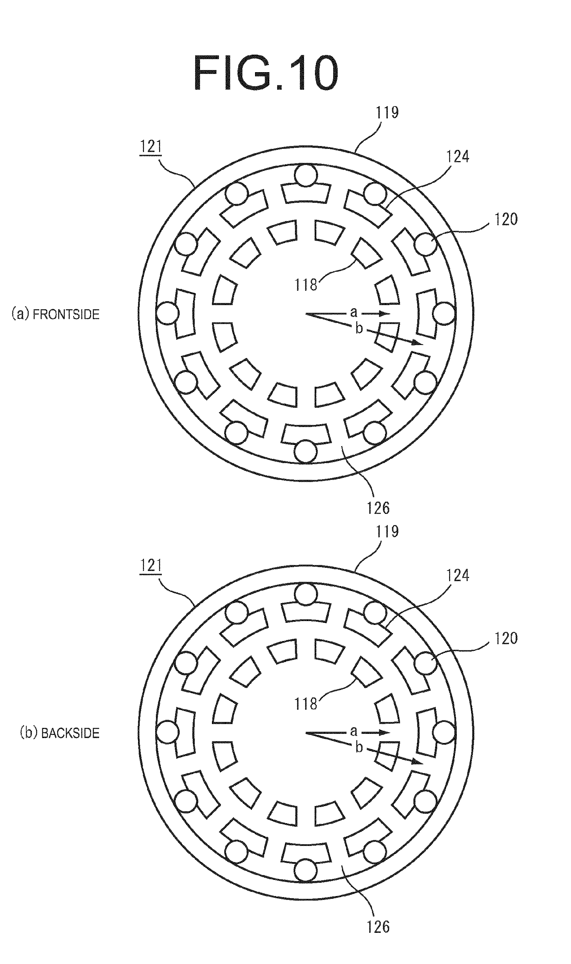

FIG. 10 (a) is a diagram showing the frontside of the stirring plate 121, and FIG. 10 (b) is a diagram showing the backside of the stirring plate 121. As shown in the figures, in the disk plate 126 forming the stirring plate 121, a plurality of protrusions 118 is formed in positions such that the spacing therebetween is a predetermined distance (for example, 2 to 3 mm), on the circle with the distance from the center being "a", on both the frontside and the backside. Further, a plurality of protrusions 124 is formed in positions such that the spacing therebetween is a predetermined distance (for example, 2 to 3 mm), on the circle with the distance from the center of the disk member 126 being "b" (in addition, b a), on both the frontside and the backside so as to respond to the spacing between protrusions 118. In the protrusions 118, 124, the frontside is brought into contact with the incurrent plate 117, and the backside is brought into contact with the excurrent plate 123. Further, a ring edge 119 that is an edge portion of the disk member 126 protrudes in the same way as the protrusions 118, 124, and is brought into contact with the incurrent plate 117 on its front side, while being brought into contact with the excurrent plate 123 on its backside. Then, on the inner side of the ring edge 119 are formed a plurality of through holes 120.

Accordingly, the ozone-mixed water 105 passes through the circular hole 116 of the incurrent plate 117 from the discharge-pipe (flexible pipe 112) side of the mixing pump 111 to enter the stirring portion 114, next passes between the protrusions 118 and 124 formed on the frontside of the stirring plate 121 to pass through the through hole 120, further passes between the protrusions 118, 124 formed on the backside of the stirring plate 121, and is discharged from the circular hole 122 of the excurrent plate 123.

In this way, herein, since used as the stirring means are the stirring portions 114 each provided with the stirring plate 121 with pluralities of protrusions 118, 124 spaced circumferentially formed, the ozone-mixed water 105 collides with the pluralities of protrusions 118, 124, and is thereby stirred, and ozone contained in the ozone-mixed water 105 is made finer. Then, the ozone-mixed water 105 passes through a plurality of stirring portions 114, stirring of the ozone-mixed water 105 is thereby repeated, and the ozone water 125 containing ozone made micro bubbles (with particle diameters ranging from 4 to 50 µm) is discharged from the last-stage stirring portion 114.

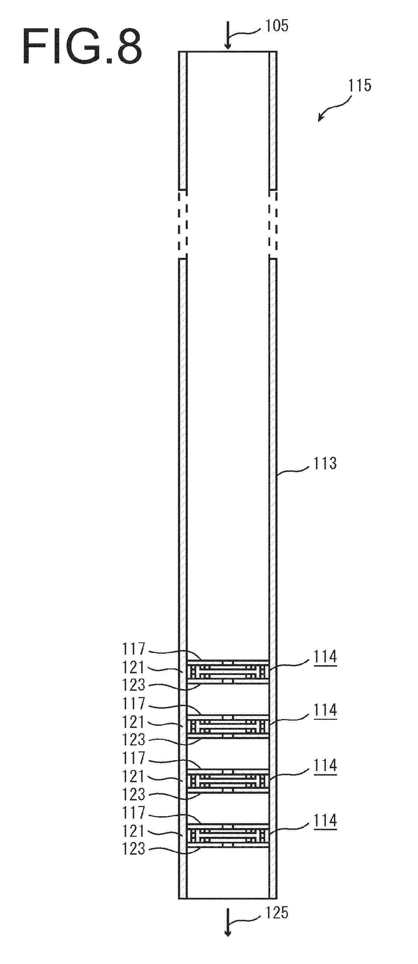

In FIG. 8, the number of installed stirring portions 114 and the installation distance between the stirring portions 114 are adjusted corresponding to the flow rate of the ozone-mixed water 105. As the number of installed stirring portions 114 is higher, or as the installation distance between the stirring portions 114 is narrower, ozone is made finer. In other words, by adjusting the running-water pressure of from the mixing pump 111 into the stirring portion 114 and the time of circulation stirring by the line mixer 115, it is possible to control the particle diameter of the ozone bubble (as the running-water pressure inside the stirring portion 114 is larger, or as the time of circulation stirring by the line mixer 115 is longer, the ozone bubble is made finer). Accordingly, by adjusting the pressure and time, it is possible to obtain the treatment-target water 104 containing many ozone bubbles with desired particle diameters (ranging from 4 to 50 µm).

In addition, it is possible to adjust the pressure inside the stirring means 114 to be larger by making the diameter of the circular hole 116 of the incurrent plate 117 larger than the diameter of the circular hole 122 of the excurrent plate 123, and on the other hand, it is possible to adjust the stirring pressure to be smaller by making the diameter of the circular hole 116 of the incurrent plate 117 smaller than the diameter of the circular hole 122 of the excurrent plate 123. In general, the pressure inside the stirring means 114 is increased by making the diameter of the circular hole 116 of the incurrent plate 117 larger than the diameter of the circular hole 122 of the excurrent plate 123.

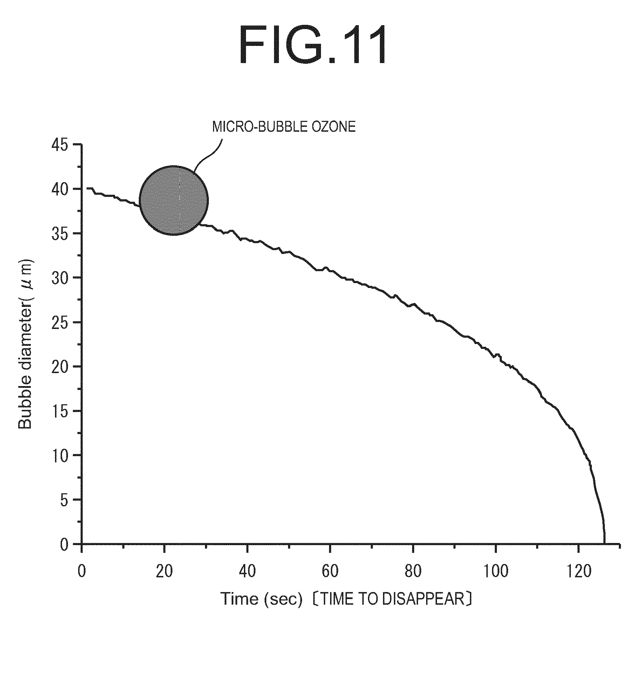

In the ozone water generating apparatus 101, by adjusting the discharge flow rate and discharge pressure of the mixing pump 111, mixing ratio between ozone and treatment-target water 104, the number of stages of each stirring means 114, positions and dimensions of the protrusions 118, 124 formed in each stirring means 114, etc. the particle diameter of ozone contained in the ozone water 125 discharged from the line mixer 115 is controlled to 4 to 50 µm. By this means, as in the graph shown in FIG. 11, it is possible to increase the duration of ozone contained in the ozone water 125.

Thus, since the duration of ozone is increased and ozone is made hard to disappear in the water, it is possible to increase the ozone density contained in the treatment-target water 104 almost in proportion to the injection time of the ozone water 125, and by this means, as compared with the conventional ozone treatment method, it is possible to oxidation-decompose organic substances and the like in the treatment-target water 104 in a short time.

In addition, herein, the power-supply Voltage and frequency output from the power-supply apparatus 107 are made adjustable, the motor 108 is made function as an inverter motor, the discharge pressure of the mixing pump 111 is made variable in the range of 3 to 8 atmospheres, the diameter of each stirring means 114 constituting the line mixer 115 is made 35 mm, the distances between the protrusions 118 and between the protrusions 124 are made 2 to 3 mm, and therefore, it is possible to make ozone contained in the ozone-mixed water 105 finer efficiently, and to adjust the particle diameter of ozone contained in the ozone water 125 to the range of 4 to 50 µm.

Further, herein, since adopted is the fine-ozone making mechanism with simplified structure for colliding the ozone-mixed water 105 with pluralities of protrusions 118, 124 formed in the stirring plate 121 constituting the stirring means 114 and thereby making ozone finer, it is possible to control the manufacturing cost of the apparatus itself to be low. Furthermore, such a problem does not occur that the ceramic filter adsorbs organic substances and is clogged when the apparatus is not operated for a long time, which occurs in the conventional ozone treatment method (for example, a method of injecting ozone into a ceramic filter or the like immersed in the treatment-target water and generating micro-bubble ozone).

Moreover, herein, the diameter of each stirring means 114 constituting the mixer 115 is made 35 mm, while the distances between the protrusions 118 and between the protrusions 124 are made 2 to 3 mm, each stirring means 114 is thereby not clogged even when a solid substance (with the size in the range of 2 to 3 mm) is contained in the treatment-target water 104 absorbed by the mixing pump 111, and therefore, even when the treatment-target water 104 stored in the water tank 103 is medical discharged water or the like, it is possible to convert the water 104 into the ozone water 125 and return to the water tank 103. Therefore, as compared with the case of mixing ozone into ordinary water to generate the ozone-mixed water 105, and injecting the water 105 into the treatment-target water 104, it is possible to increase the ozone density in the treatment-target water 104 to the practical density in a short time without increasing the water amount of the treatment-target water 104 stored in the water tank 103.

Accordingly, the apparatus is optimal in cleaning, sterilization or disinfection treatment of medical instruments and equipment used in the surgery, treatment, medical care or the like in medical institutions such as a hospital. In other words, by bringing a medical instrument such as a surgical knife used in the surgery, treatment, medical care or the like into contact for a predetermined time, it is possible to sterilize or disinfect the medical instrument itself.

### «Modification of the zone water generating apparatus»

The above-mentioned ozone water generating apparatus 101 stirs the ozone-mixed water 105 discharged from the mixing pump 111 by the line mixer having a plurality of stirring portions 114, and thereby generates the ozone water 125 containing ozone made micro bubbles (with particle diameters ranging from 4 to 50 µm). As a substitute therefor, for example, it may be adopted putting a ceramic filter or the like into the treatment-target water 104, and supplying ozone generated by the ozone supply apparatus 102 to the ceramic filter to generate ozone with particle diameters ranging from 4 to 50 µm.

Further, it may be adopted stirring the ozone-mixed water 105 discharged from the mixing pump 111 strongly by a propeller or the like to make ozone contained in the ozone-mixed water 105 finer, and generating ozone with particle diameters ranging from 4 to 50 µm.

Furthermore, stirring means comprised of a cylindrical stirring block may be used, as a substitute for the above-mentioned stirring means 114.

### (Example of the stirring block I)

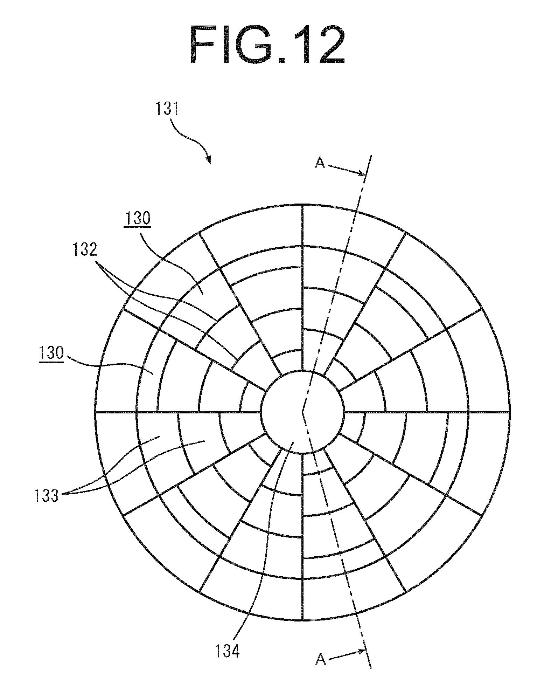

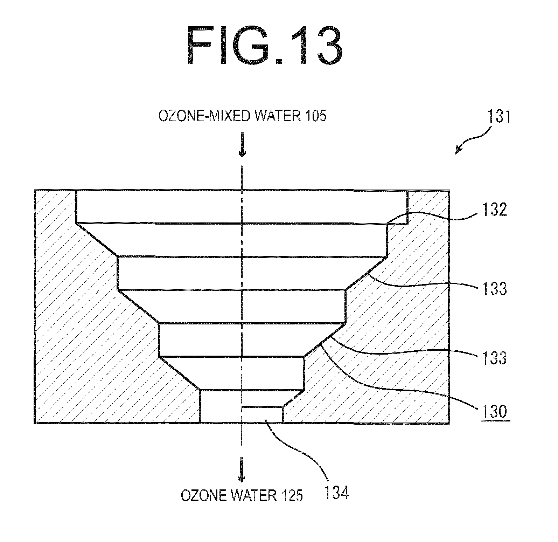

FIG. 12 is a front view of stirring means (stirring block) 131, and FIG. 13 is a sectional view taken along the line A-A in FIG. 12.

As shown in the figures, the stirring block 131 is comprised of a block substantially in the shape of a cylinder having a hole 134 in the center. On the periphery of the hole 134 in the center, as shown in FIG. 13, step heights forming a plurality of protrusions are spaced circumferentially and formed. Shown herein is the example comprised of 12 stirring surfaces 130 divided on a 30-degree basis.

Among the stirring surfaces 130, in six stirring surfaces 130 disposed at 60-degree intervals, on the oblique surface formed so that the surface approaches the center line of the block 131 as the surface comes closer to each block front end side (lower end side in FIG. 13), a plurality of cylindrical portions 132 and a plurality of taper portions 133 are formed in the order of cylindrical portion 132→taper (oblique surface) portion 133→cylindrical portion 132→taper portion 133 cylindrical portion 132→taper portion 133. Further, in six stirring surfaces 130 disposed between the stirring surfaces 130, on the oblique surface formed so that the surface approaches the center line of the block 131 as the surface comes closer to each block front end side (lower end side in FIG. 13), a plurality of cylindrical portions 132 and a plurality of taper portions 133 are formed in the order of taper portion 133→cylindrical portion 132→taper portion 133→cylindrical portion 132→taper portion 133→cylindrical portion 132. In other words, positions of each cylindrical portion 132 and each taper portion 133 are determined so that the taper portions 133 formed on remaining six stirring surfaces 130 are disposed between cylindrical portions 132 formed on six stirring surfaces 130 disposed at 60-degree intervals.

Such a stirring block 131 is brought into intimate contact and disposed with/in the hollow pipe 113 so that the ozone-mixed water 105 flows from the larger diameter side to the smaller diameter size of the hole 134. Then, in the running-water channel, many vortexes occur in the ozone-mixed water 105 to stir the water 105 by the interaction between the cylindrical portion 132 and taper portion 133 of each stirring surface 130, and micro-bubble ozone (with particle diameters ranging from 4 to 50 µm) is generated. In other words, also in the stirring means (stirring block) 131 as described above, as in the above-mentioned stirring means 114, the ozone-mixed water 105 supplied from the mixing pump 111 collides sequentially with a plurality of protrusions with the running-water pressure applied, in the enclosed running-water channel in which a plurality of step-shaped protrusions is disposed in the conical inner surface (stirring surface 130), ozone contained in the ozone-mixed water 105 is thereby made finer, and it is possible to generate the ozone water 125.

At this point, as an example, on the stirring surface 130, step-shaped protrusions are formed in increments of 4 mm in the axial direction (the vertical direction in FIG. 13) of the stirring block 131 and in increments of 10 mm in the diameter in the circumferential direction (the horizontal direction in FIG. 13), and the hole 134 is formed in the diameter of 10 mm. By this means, even when the treatment-target water 104 absorbed by the mixing pump 111 contains a solid substance (solid substance with the size of 10 mm or less), the block is not clogged.

The stirring block 131 in such a shape can be manufactured with ease only by once injection-forming plastic material, and therefore, can be manufactured at lower cost than the stirring portion 114 comprised of the incurrent plate 117, stirring plate 121 and excurrent plate 123.

### (Example of the stirring block II)

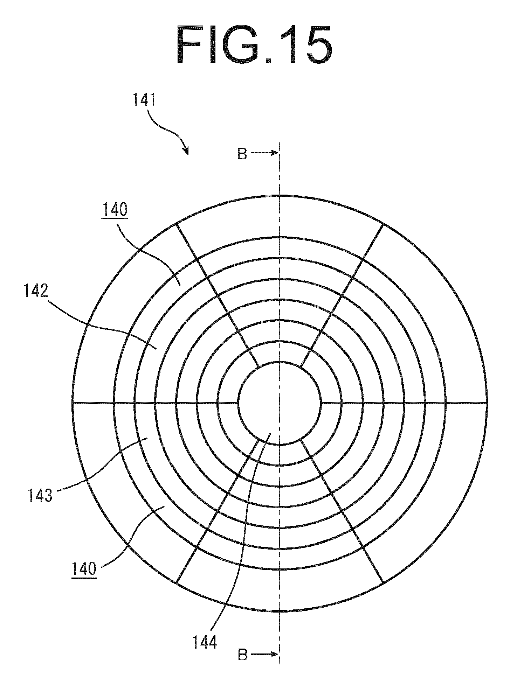

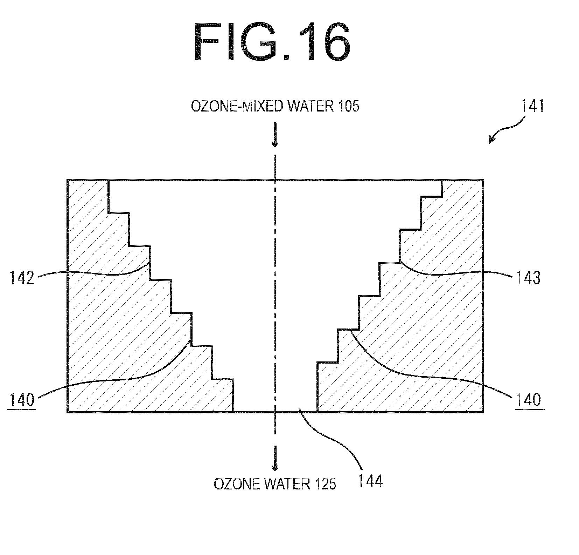

FIG. 15 is a front view of stirring means (stirring block) 141, and FIG. 16 is a sectional view taken along the line B-B in FIG. 12.

As shown in the figures, the stirring block 141 is comprised of a block substantially in the shape of a cylinder having a hole 144 in the center. On the periphery of the hole 144 in the center, as shown in FIG. 16, step heights forming a plurality of protrusions are spaced circumferentially and formed. Shown herein is the example comprised of 6 stirring surfaces 140 divided on a 60-degree basis.

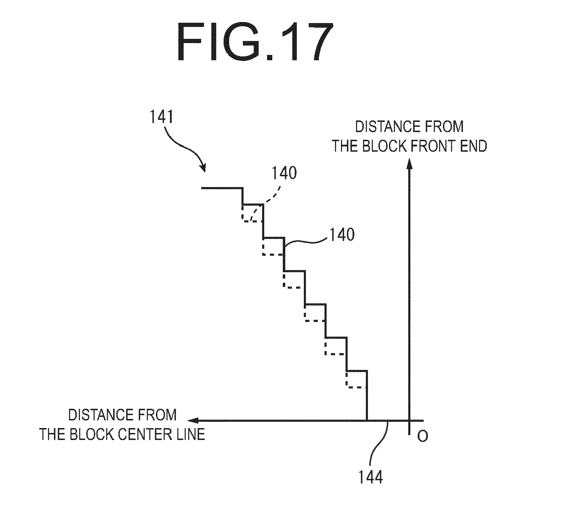

Among the stirring surfaces 140, in three stirring surfaces 140 disposed at 120-degree intervals, step-shaped step height portions 142 are formed so that the surface approaches the center line of the block 141 as the surface comes closer to each block front end side (lower end side in FIG. 16). Also in the other three stirring surfaces 140 disposed between the stirring surfaces 140, step-shaped step height portions 143 are formed so that the surface approaches the center line of the block 141 as the surface comes closer to each block front end side (lower end side in FIG. 16). As shown in FIGs. 16 and 17, the horizontal surface and the perpendicular surface of the step height portion 143 and the horizontal surface and the perpendicular surface of the step height portion 142 of the adjacent stirring surface 140 are disposed in a staggered configuration.

Such a stirring block 141 is brought into intimate contact and disposed with/in the hollow pipe 113 so that the ozone-mixed water 105 flows from the larger diameter side to the smaller diameter size of the hole 144. Then in the running-water channel, many vortexes occur in the ozone-mixed water 105 to stir the water 105 by the interaction between the step height portions 142 and 143 of each stirring surface 140, and micro-bubble ozone (with particle diameters ranging from 4 to 50 µm) is generated. In other words, also in the stirring means (stirring block) 141 as described above, as in the above-mentioned stirring means 114, the ozone-mixed water 105 supplied from the mixing pump 111 collides sequentially with a plurality of protrusions with the running-water pressure applied, in the enclosed running-water channel in which a plurality of step-shaped protrusions is disposed in the conical inner surface (stirring surface 140), ozone contained in the ozone-mixed water 105 is thereby made finer, and it is possible to generate the ozone water 125.

At this point, as an example, on the stirring surface 140, step-shaped protrusions are formed in increments of 4 mm in the axial direction (the vertical direction in FIG. 16) of the stirring block 141 and in increments of 5 mm in the diameter in the circumferential direction (the horizontal direction in FIG. 16), and the hole 144 is formed in the diameter of 10 mm. By this means, even when the treatment-target water 104 absorbed by the mixing pump 111 contains a solid substance (solid substance with the size of 10 mm or less), the block is not clogged.

The stirring block 141 in such a shape can be manufactured with ease only by once injection-forming plastic material, and therefore, can be manufactured at lower cost than the stirring portion 114 comprised of the incurrent plate 117, stirring plate 121 and excurrent plate 123.

### «One Embodiment of the cleaning sterilization apparatus according to the invention»

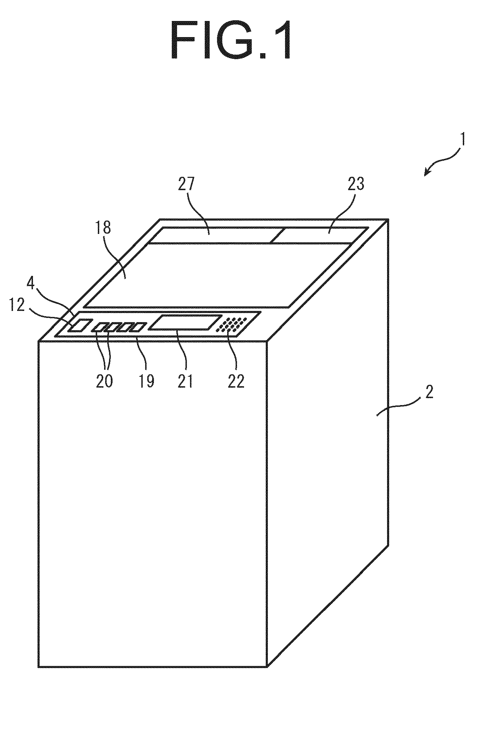

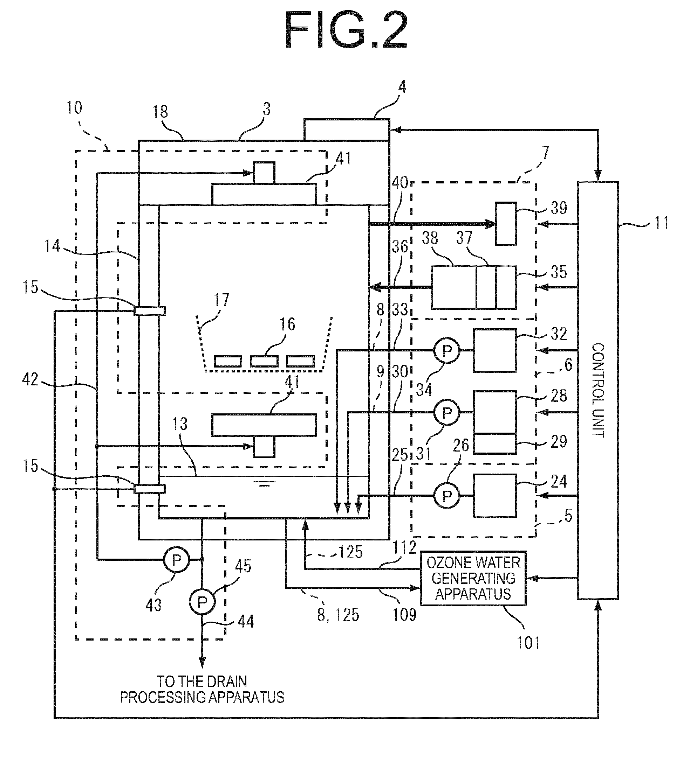

FIG. 1 is an appearance diagram showing an Embodiment of the cleaning sterilization apparatus according to the invention using the above-mentioned ozone water generating apparatus 101. Further, FIG. 2 is a functional block diagram of the apparatus. In addition, in the figures, portions corresponding to parts in FIG. 5 are assigned the same reference numerals.

The cleaning sterilization apparatus 1 according to the invention is provided with an apparatus housing 2, a cleaning and sterilizing unit 3 disposed inside the apparatus housing 2 to clean and sterilize a used medical instrument (object to be cleaned) 16 (see FIG. 2), an operating panel 4 disposed on the apparatus housing 2 to be operated by a user, a cleaning agent supply unit 5 disposed inside the apparatus housing 2 to supply a cleaning agent to the cleaning and sterilizing unit 3, a hot water/cold water supply unit 6 disposed inside the apparatus housing 2 to supply hot water 9 and cold water 8 to the cleaning and sterilizing unit 3, a dry unit 7 disposed inside the apparatus housing 2 to supply hot air and cold air to the cleaning and sterilizing unit 3 while exhausting air and drying the used medical instrument, the ozone water generating apparatus 101 disposed inside the apparatus housing 2 to make cold water (that corresponds to the treatment-target water 104) 8 stored in the cleaning and sterilizing unit 3 the ozone water 125, a squirt/drain unit 10 disposed inside the apparatus housing 2 to take in the hot water 9, cleaning water 13 or the like stored in the cleaning and sterilizing unit 3 to inject into the cleaning and sterilizing unit 3, and a control unit 11 that controls the cleaning agent supply unit 5 to squirt/drain unit 10 corresponding to directions from the operating panel 4.

In using the cleaning sterilization apparatus 1, first, a user opens a cover 18 of the cleaning and sterilizing unit 3, sets a basket 17 (see FIG. 2) with used medical instruments 16 therein into the cleaning and sterilizing unit 3, and presses a cleaning and sterilizing start button 12 of the operating panel 4. Then, the cleaning sterilization apparatus 1 sequentially performs cleaning treatment for squirting the cleaning water 13 that is a mixture of the cleaning agent and hot water 9 to the medical instruments 16, rinsing treatment for squirting the hot water 9, sterilization treatment for squirting the ozone water 125 or immersing in the ozone water 125, and dry treatment for blowing hot air and cold air, and when finishing, notifies that cleaning and sterilization of the medical instruments 16 is completed using a buzzer sound or the like.

The cleaning and sterilizing unit 3 is provided with a cleaning and sterilizing container (that corresponds to the water tank 103 in FIG. 5) 14 in which cleaning and sterilizing operation is performed on the medical instruments 16, the cover 18 to enable the cleaning and sterilizing container 14 to be sealed, and a plurality of water level sensors disposed inside the cleaning and sterilizing container 14. Then, in cleaning and sterilizing the used medical instruments 16, the basket 17 storing the used medical instruments 16 is set into the cleaning and sterilizing container 14. In addition, detection results of water levels of the hot water 9, cold water 8, ozone water 125 and the like by each water level sensor 15 are supplied to the control unit 11.

The operating panel 4 is provided with a panel body 19, and the cleaning and sterilizing start button 12, various setting buttons 20 operated in adjusting the cleaning time, rinsing time, ozone sterilization time and the like, a display 21 that displays the treatment content or the like, and a buzzer 22 that generates a buzzer sound, each provided in the panel body 19. Then, when each setting button 20 is operated, corresponding to the operation content, directions for adjusting the cleaning time, rinsing time, ozone sterilization time and the like are generated and supplied to the control unit 11. Further, when the cleaning and sterilizing start button 12 is operated, cleaning and sterilizing start directions are generated and supplied to the control unit 11. Furthermore, when display directions, buzzer sound output directions or the like are supplied from the control unit 11, the panel 4 displays the directed content on the display 21, or causes the buzzer 22 to output a buzzer sound.

The cleaning agent supply unit 5 is provided with a cleaning agent container 24 that holds a cleaning agent which is filled while a cover 23 (see FIG. 1) provided in the apparatus housing 2 is opened, a pipe 25 that connects between a cleaning agent supply opening provided in the cleaning and sterilizing container 14 and the cleaning agent container 24, and a cleaning agent supply pump 26 which is inserted in some midpoint in the pipe 25 and is turned on/off based on directions from the control unit 11. Then, when the control unit 11 supplies cleaning agent supply directions, the unit 5 turns on the cleaning agent supply pump 26, and supplies the cleaning agent into the cleaning and sterilizing container 14 in the path of the cleaning agent container 24→pipe 25→cleaning agent supply pump 26→pipe 25→cleaning and sterilizing container 14.

The hot water/cold water supply unit 6 is provided with a hot water container 28 that stores water which is filled while a cover 27 (see FIG. 1) is opened, a thermostat 29 that applies power to a heater to warm water stored inside the hot water container 28 and make the hot water 9 with a designated temperature when the control unit 11 supplies hot water generation directions, a pipe 30 that connects between a hot water opening formed in the cleaning and sterilizing container 14 and the hot water container 28, a hot water supply pump 31 which is inserted in some midpoint in the pipe 30 and is turned on/off based on directions from the control unit 11, a cold water container 32 that stores the cold water 8 which is filled while the cover 27 is opened, a pipe 33 that connects between a cold water opening formed in the cleaning and sterilizing container 14 and the cold water container 32, and a cold water supply pump 34 which is inserted in some midpoint in the pipe 33 and is turned on/off based on directions from the control unit 11. Then, when the control unit 11 outputs hot water generation directions, the unit 6 heats water stored in the hot water container 28 by the thermostat 29, and makes the hot water 9 with the designated temperature. Further, when the control unit 11 outputs hot water supply directions, the unit 6 turns on the hot water supply pump 31, and supplies the hot water 9 into the cleaning and sterilizing container 14 in the path of the hot water container 28→pipe 30→hot water supply pump 31→pipe 30→hot water opening of the cleaning and sterilizing container 14. Furthermore, when the control unit 11 outputs cold water supply directions, the unit 6 turns on the cold water supply pump 34, and supplies the cold water 8 into the cleaning and sterilizing container 14 in the path of the cold water container 32→pipe 33→cold water supply pump 34→pipe 33→cold water opening of the cleaning and sterilizing container 14.

The dry unit 7 is provided with an intake shutter 35 which is attached to an intake opening formed in the apparatus housing 2 and is turned on/off based on directions from the control unit 11, an intake duct 36 that connects between the intake opening formed in the apparatus housing 2 and a hot air/cold air discharge opening formed in the cleaning and sterilizing container 14, an intake fan mechanism 37 which is inserted in some midpoint in the intake duct 36 and is turned on/off based on directions from the control unit 11, a heater 38 which is inserted in some midpoint in the intake duct 36 and is turned on/off based on directions from the control unit 11, an exhaust shutter 39 which is attached to an exhaust opening formed in the apparatus housing 2 and is turned on/off based on directions from the control unit 11, and an exhaust duct 40 that connects between the exhaust opening formed in the apparatus housing 2 and a hot air/cold air intake opening formed in the cleaning and sterilizing container 14.

Then, when the control unit 11 outputs hot air directions, the unit 7 opens the intake shutter 35 and exhaust shutter 39, while turning on the intake fan mechanism 37 and heater 38, guides air taken in from the outside of the apparatus to the heater 38 in the path of the intake opening of the apparatus housing 2→intake shutter 35→intake fan mechanism 37→heater 38, makes the hot air with the designated temperature, and then, supplies the hot air into the cleaning and sterilizing container 14 in the path of the heater 38→intake duct 36→hot air/cold air discharge opening of the cleaning and sterilizing container 14, Further, in parallel with the operation, the unit 7 discharges the hot air inside the cleaning and sterilizing container 14 to the outside of the apparatus in the path of the hot air/cold air intake opening of the cleaning and sterilizing container 14→exhaust duct 40→exhaust shutter 39→exhaust opening of the apparatus housing 2.

Meanwhile, when the control unit 11 outputs cold air directions, the unit 7 opens the intake shutter 35 and exhaust shutter 39, while turning on the intake fan mechanism 37, and supplies the cold air into the cleaning and sterilizing container 14 in the path of the intake opening of the apparatus housing 2→intake shutter 35→intake fan mechanism 37→heater 38→intake duct 36→hot air/cold air discharge opening of the cleaning and sterilizing container 14. Further, in parallel with the operation, the unit 7 discharges the cold air inside the cleaning and sterilizing container 14 to the outside of the apparatus in the path of the hot air/cold air intake opening of the cleaning and sterilizing container 14→exhaust duct 40→exhaust shutter 39→exhaust opening of the apparatus housing 2.

The ozone water generating apparatus 101 is disposed inside the apparatus housing 2, and when the control unit 11 outputs ozone water generation directions, takes in water (cold water 8) stored in the cleaning and sterilizing container 14 by the flexible pipe 109, while mixing and stirring the cold water 8 and ozone. Then, the apparatus 101 generates the ozone water 125 containing ozone made micro bubbles (with particle diameters ranging from 4 to 50 µm), and then, supplies the ozone water 125 to the cleaning and sterilizing container 14 through the flexible pipe 112.

The squirt/drain unit 10 is provided with two rotating nozzles 41 respectively disposed in positions with the basket 17 therebetween in the cleaning and sterilizing container 14, a pipe 42 that connects between each of the rotating nozzles 41 and an intake opening formed on the bottom of the cleaning and sterilizing container 14, a circulating pump 43 which is inserted in some midpoint in the pipe 42 and is turned on/off based on directions from the control unit 11, a drain pipe 44 that connects between the intake opening formed on the bottom of the cleaning and sterilizing container 14 and a drain processing apparatus (omitted in the figure) disposed outside the apparatus, and a drain pump 45 which is disposed in some midpoint in the drain pipe 44 and is turned on/off based on directions from the control unit 11. Then, when the control unit 11 outputs squirt directions, the unit 10 turns on the circulating pump 43, and guides the cleaning water 13, hot water 9 and the like present inside the cleaning and sterilizing container 14 to each rotating nozzle 41 to squirt, in the path of the intake opening of the cleaning and sterilizing container 14→pipe 42→circulating pump 43→pipe 42→each rotating nozzle 41. Meanwhile, when the control unit 11 outputs drain instructions, the unit 10 turns on the drain pump 45, and supplies the cleaning water 13, hot water 9, ozone water 125 and the like stored on the bottom inside the cleaning and sterilizing container 14 to the drain processing apparatus to cause the apparatus to perform drain processing, in the path of the intake opening of the cleaning and sterilizing container 14→drain pipe 44→drain pump 45→drain pipe 44→the drain processing apparatus disposed outside the apparatus.

The control unit 11 is provided with a processing substrate with a CPU for performing various kinds of processing and the like mounted thereon, and signal cables that connect between a connector of the processing substrate and a connector of each of the cleaning and sterilizing unit 3 to squirt/drain unit 10. Then, when each setting button 20 of the operating panel 4 is operated and adjustment directions are output from the operating panel 4, the unit 11 retrieves the directions and changes the cleaning time, rinsing time, ozone sterilization time and the like stored in the storage apparatus. Further, when the basket 17 with the used medical instruments 16 and the like put therein is set on the inside of the cleaning and sterilizing container 14, and the cleaning and sterilizing start button 12 of the operating panel 4 is pressed with the cover 18 closed, the unit 11 sequentially performs cleaning treatment for squirting the cleaning water 13 obtained by mixing the cleaning agent and the hot water 9 to the medical instruments 16 put in the cleaning and sterilizing container 14, rinsing treatment for squirting the hot water 9 to the instruments 16, sterilization treatment for squirting the ozone water 125 or immersing in the ozone water 125, and dry treatment for blowing hot air and cold air. Then, when a series of such treatments is finished, the unit 11 outputs display directions, buzzer sound output directions and the like, and displays a message indicative of completion of cleaning and sterilization on the display 21, while outputting a busser sound from the buzzer 22 to inform the user of completion of the cleaning and sterilization of the medical instruments 16.

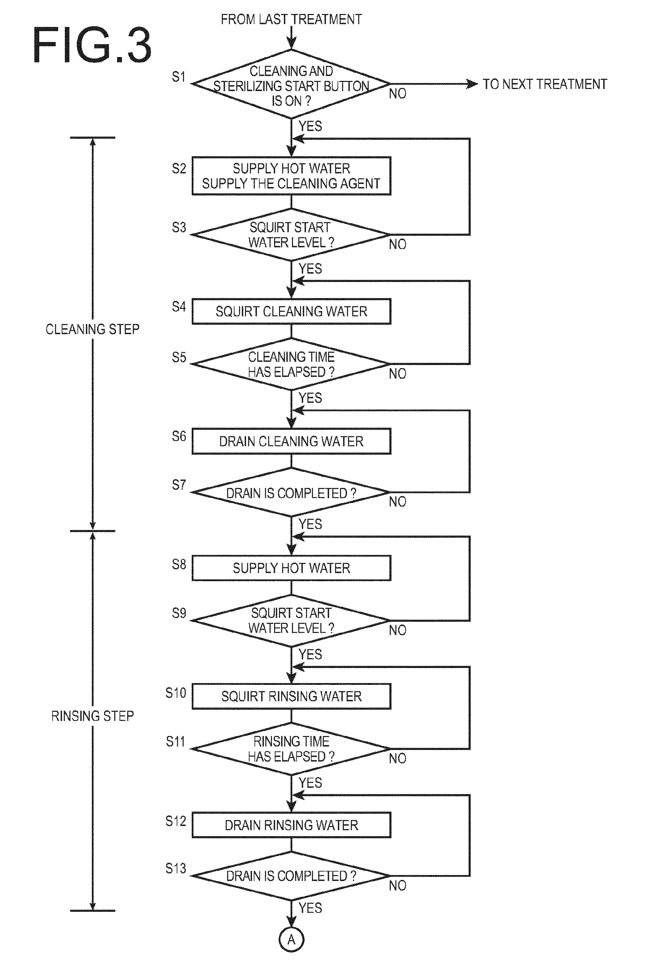

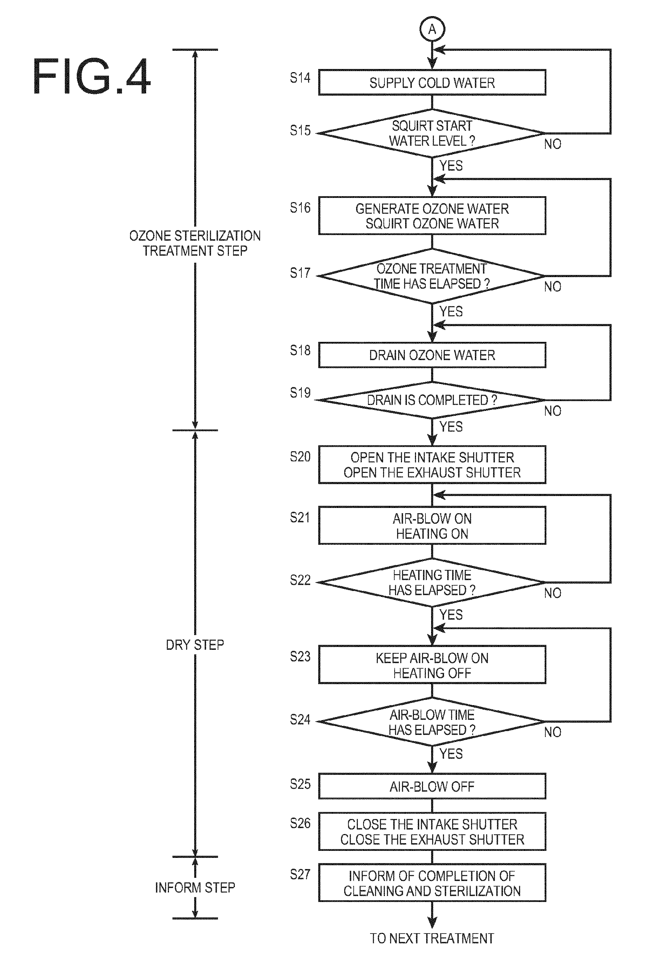

The operation of the cleaning sterilization apparatus 1 will be described below with reference to timing charts as shown in FIGs. 3 and 4. In addition, as an example, the following description describes the case of squirting the ozone water 125 to the used medical instrument 16 to perform sterilization treatment.

First, the user opens the cover 18 of the cleaning and sterilizing unit 3, sets the basket 17 with used medical instruments 16 put therein into the cleaning and sterilizing container 14, closes the cover 18, and presses the cleaning and sterilizing start button 12 on the operating panel 4. When the cleaning and sterilizing start button 12 is pressed (step S1), the control unit 11 outputs cleaning agent supply directions and hot water supply directions, and the cleaning agent supply unit 5 supplies a cleaning agent into the cleaning and sterilizing container 14, while the hot water/cold water supply unit 6 supplies hot water 9 into the cleaning and sterilizing container 14 (step S2).

Then, when mixed water (cleaning water 13) of the cleaning agent and hot water 9 stored in the cleaning and sterilizing container 14 becomes a certain water level and a detection result indicative of a cleaning start water level being obtained is output from each water level sensor 15 provided in the cleaning sterilizing container 14, the control unit 11 outputs cleaning agent supply halt directions and hot water supply halt directions, and halts cleaning agent supply by the cleaning agent supply unit 5, while haling hot water supply by the hot water/cold water supply unit 6 (step S3).

Next, the control unit 11 outputs squirt directions, turns on the circulating pump 43 of the squirt/drain unit 10, and supplies the cleaning water 13 stored inside the cleaning and sterilizing container 14 to each rotating nozzle 41 in the path of the inside of the cleaning and sterilizing container 14→pipe 42→circulating pump 43→pipe 42→each rotating nozzle 41. By this means, each rotating nozzle 41 squirts the cleaning water 13. Each rotating nozzle 41 rotates by the reaction force caused at this point, and the medical instruments 16 held in the basket 17 inside the cleaning and sterilizing container 14 are cleaned by the squirted cleaning water 13. The cleaning water 13 finishing cleaning is returned to the bottom inside the cleaning and sterilizing container 14.

Hereinafter, until beforehand set cleaning time has elapsed, the circulating pump 43 of the squirt/drain unit 10 is kept on, the cleaning water 13 that is returned to the bottom inside the cleaning and sterilizing container 14 is continuously supplied to each rotating nozzle 41, and the aforementioned cleaning treatment of the medical instruments 16 is continued (step S4).

Next, when the beforehand set cleaning time has elapsed (step S5), the control unit 11 outputs squirt halt directions, returns the circulating pump 43 of the squirt/drain unit 10 to the off state, and finishes cleaning of the medical instruments 16 by the cleaning water 13. Further, the control unit 11 outputs drain directions, turns on the drain pump 45 of the squirt/drain unit 10, and discharges the cleaning water 13 stored on the bottom inside the cleaning and sterilizing container 14 to the outside of the apparatus, and the drain processing is performed in the drain processing apparatus (step S6).

Next, when all the cleaning water 13 present inside the cleaning and sterilizing container 14 is discharged and each water level sensor 15 provided in the cleaning and sterilizing container 14 outputs a detection result indicating that the drain of the cleaning water 13 has been completed (step S7), the control unit 11 outputs hot water supply directions, and the hot water/cold water supply unit 6 supplies the hot water 9 into the cleaning and sterilizing container 14 (step S8).

Then, when the hot water 9 stored in the cleaning and sterilizing container 14 becomes a certain water level and each water level sensor 15 provided in the cleaning and sterilizing container 14 outputs a detection result indicative of a rinsing start water level being obtained, the control unit 11 outputs hot water supply halt directions, and halts hot water supply by the hot water/cold water supply unit 6. Further, the control unit 11 outputs squirt directions, turns on the circulating pump 43 of the squirt/drain unit 10, and supplies the hot water 9 stored in the cleaning and sterilizing container 14 to each rotating nozzle 41 in the path of the inside of the cleaning and sterilizing container 14→pipe 42→circulating pump 43→pipe 42→each rotating nozzle 41 (step S9).

By this means, each rotating nozzle 41 squirts the hot water 9, and rotates by the reaction force caused at this point. Then, the medical instruments 16 held in the basket 17 inside the cleaning and sterilizing container 14 are rinsed by the hot water 9 squirted from each rotating nozzle 41, and the hot water 9 finishing rinsing is returned to the bottom inside the cleaning and sterilizing container 14.

Hereinafter, until beforehand set rinsing time has elapsed, the circulating pump 43 of the squirt/drain unit 10 is kept on, the hot water 9 that is returned to the bottom inside the cleaning and sterilizing container 14 is continuously supplied to each rotating nozzle 41, and the aforementioned rinsing treatment of the medical instruments 16 is continued (step S10).

Next, when the beforehand set rinsing time has elapsed (step S11), the control unit 11 outputs squirt halt directions, returns the circulating pump 43 of the squirt/drain unit 10 to the off state, and finishes rinsing of the medical instruments 16 by the hot water 9. Further, the control unit 11 outputs drain directions, turns on the drain pump 44 of the squirt/drain unit 10, and discharges the hot water 9 stored on the bottom inside the cleaning and sterilizing container 14 to the outside of the apparatus, and the drain processing is performed in the drain processing apparatus (step S12).

Subsequently, when all the hot water 9 inside the cleaning and sterilizing container 14 is discharged and each water level sensor 15 provided in the cleaning and sterilizing container 14 outputs a detection result indicating that the drain of the hot water 9 has been completed (step S13), the control unit 11 outputs cold water supply directions, and the hot water/cold water supply unit 6 supplies the cold water 8 into the cleaning and sterilizing container 14 (step S14).

Then, when the cold water 8 stored in the cleaning and sterilizing container 14 becomes a certain water level and each water level sensor 15 provided in the cleaning and sterilizing container 14 outputs a detection result indicative of an ozone sterilization treatment start water level being obtained, the control unit 11 outputs cold water supply halt directions, and halts cold water supply by the hot water/cold water supply unit 6. Further, the control unit 11 outputs ozone-mixed water generation directions, and turns on the ozone water generating apparatus 101 (step S15).

By this means, the ozone water generating apparatus 101 takes in water (cold water 8) stored in the cleaning and sterilizing container 14, mixes and stirs the cold water 8 and ozone, and generates the ozone water 125 containing ozone made micro bubbles (with particle diameters ranging from 4 to 50 µm). Further, the control unit 11 outputs squirt directions, turns on the circulating pump 43 of the squirt/drain unit 10, and supplies the ozone water 125 stored inside the cleaning and sterilizing container 14 to each rotating nozzle 41 in the path of the inside of the cleaning and sterilizing container 14→pipe 42→circulating pump 43→pipe 42→each rotating nozzle 41.

By this means, each rotating nozzle 41 squirts the ozone water 125, and rotates by the reaction force caused at this point. Then, the medical instruments 16 held in the basket 17 inside the cleaning and sterilizing container 14 are ozone-sterilizedby the ozone water 125 squirted from each rotating nozzle 41, and the ozone water 125 is returned to the bottom inside the cleaning and sterilizing container 14.

Hereinafter, until beforehand set ozone sterilization treatment time has elapsed, the generation processing of the ozone water 125 by the ozone water generating apparatus 101 is repeated, and the ozone concentration of the ozone water 125 is increased. Concurrently therewith, the circulating pump 43 of the squirt/drain unit 10 is kept on, the ozone water 125 that is returned to the bottom inside the cleaning and sterilizing container 14 is continuously circulated and supplied to each rotating nozzle 41, and the aforementioned ozone sterilization treatment of the medical instruments 16 is continued (step S16).

Next, when the beforehand set ozone sterilization treatment time has elapsed (step S17), the control unit 11 outputs ozone generation halt directions, and finishes the generation processing of the ozone water 125 by the ozone water generating apparatus 101. Further, the control unit 11 outputs squirt halt directions, and returns the circulating pump 43 of the squirt/drain unit 10 to the off state. Furthermore, the control unit 11 outputs drain directions, turns on the drain pump 44 of the squirt/drain unit 10, and starts the drain of the ozone water 125 stored on the bottom inside the cleaning and sterilizing container 14 (step S18).

Subsequently, when all the ozone water 125 present inside the cleaning and sterilizing container 14 is discharged and each water level sensor 15 provided in the cleaning and sterilizing container 14 outputs a detection result indicating that the drain of the ozone water 125 has been completed (step S19), the control unit 11 outputs hot air supply directions, and opens the intake shutter 35 and exhaust shutter 39 of the dry unit 7. Further, the unit 11 turns on the intake fan mechanism 37 and heater 38, blows hot air into the cleaning and sterilizing container 14, and dries the medical instruments 16 put in the basket 17. In addition, the hot air containing moisture present inside the cleaning and sterilizing container 14 is exhausted to the outside of the apparatus (step S20).

Hereinafter, until beforehand set hot air dry time has elapsed, with the intake shutter 35 and exhaust shutter 39 of the dry unit 7 opened, the intake fan mechanism 37 and heater 38 are kept on, the hot air is blown into the cleaning and sterilizing container 14 while continuing exhaust, and the medical instruments 16 put in the basket 17 are dried (step S21).

Next, when the beforehand set hot air dry time has elapsed (step S22), the control unit 11 outputs hot air supply halt directions and cold air supply directions, returns the heater 38 to the off state while keeping the intake fan mechanism 37 at the on state, and blows cold air into the cleaning and sterilizing container 14. By this means, the temperatures of the inner wall of the cleaning and sterilizing container 14 and medical instruments 16 put in the basket 17 are decreased, while the cold air containing heat present inside the cleaning and sterilizing container 14 is exhausted to the outside of the apparatus (step S23).

Then, when beforehand set cold air time has elapsed, and the temperatures of the cleaning and sterilizing container 14 and medical instruments 16 put in the basket 17 become a predetermined temperature or less (step S24), the control unit 11 outputs cold air supply halt directions, returns the intake fan mechanism 37 to the off state, closes the intake shutter 35 and exhaust shutter 39, and returns the inside of the cleaning and sterilizing container 14 to the sealed state (steps S25, S26).

Subsequently, the control unit 11 outputs cleaning and sterilization finish directions, display data/buzzer sound output directions, etc. displays a cleaning and sterilization treatment completion message on the operating panel 4 only for a certain time, while outputting a buzzer sound indicative of finish of cleaning and sterilization from the buzzer 22, and notifies that cleaning and sterilization of the medical instruments 16 is completed (step S27).

Thus, in this Embodiment, the generated ozone water 125 is squirted to the medical instruments 16, while the squirted ozone water 125 is stored, ozone made micro bubbles by the ozone generating apparatus 101 is further contained in the ozone water 125, and the ozone water 125 is squirted repeatedly. In this way, the ozone water 125 containing micro-bubble ozone is squirted while being circulated, the ozone concentration is enhanced to increase the sterilization ability, and it is made possible to perform efficient sterilization treatment in a short time.

### «Another Embodiment»

In addition, the aforementioned Embodiment describes the case of squirting the ozone water 125 to used medical instruments 16 and performing sterilization treatment, and instead of squirting the ozone water 125, the medical instruments 16 may be immersed in the ozone water 125 to perform sterilization treatment. In this case, the cold water 8 is stored in the cleaning and sterilizing container 14 to a water level such that the medical instruments 16 are fully immersed. Then, the ozone water generating apparatus 101 takes in the cold water 8, mixes and stirs the cold water 8 and ozone, generates the ozone water 125 containing ozone made micro bubbles (with particle diameters ranging from 4 to 50 µm), and supplies into the cleaning and sterilizing container 14, and the ozone sterilization treatment is performed. Absorption of the cold water 8 (ozone water 125) and generation and supply of the ozone water 125 is continued over beforehand set ozone sterilization treatment time, and the ozone water 125 is circulated at a predetermined flow rate. By this means, the ozone concentration of the ozone water 125 containing micro-bubble ozone is enhanced to increase the sterilization ability, and it is made possible to perform efficient sterilization treatment in a short time.

Further, the above-mentioned Embodiment shows the example of providing the cleaning step (or rinsing step) and the ozone sterilization treatment step separately, but by the ozone water generating apparatus 101 taking in the cleaning water 13 (or hot water 9) used in the cleaning step (or rinsing step) and mixing and stirring the micro-bubble ozone, the cleaning step (or rinsing step) and the ozone sterilization treatment step may be performed at a time. In this case, the cleaning step (or rinsing step) that is performed concurrently with the ozone sterilization treatment step may be performed by squirting as in the above-mentioned Embodiment or immersing.

Furthermore, in the invention, in addition to the configuration as shown in the above-mentioned Embodiment, the cleaning and sterilizing container 14 may be further provided at its inside with an ultrasonic transducer (ultrasonic vibration means). The ultrasonic transducer is provided with a piezoelectric element, and is disposed so that the vibration surface is exposed to the inside of the cleaning and sterilizing container 14 and directly comes into contact with the liquid such as the ozone water 125 and cleaning water 13, or comes into intimate contact with the bottom of the cleaning and sterilizing container 14. When the piezoelectric element is actuated, ultrasonic vibration is conveyed through the liquid, and suspends stains adhering to the surfaces of the used medical instruments 16 to remove. Such removal of stains by ultrasonic wave may be performed in the cleaning step, or performed in the ozone sterilization treatment step. Alternately, as described above, the removal may be performed in the cleaning step (or rinsing step) that is performed concurrently with the ozone sterilization treatment step. In this case, to exploit the maximum cleaning effect by ultrasonic wave, it is suitable that the aforementioned step is performed by immersion. In this way, by adding the ultrasonic vibration means, it is possible to increase cleaning sterilization power of the cleaning sterilization apparatus 1.

In addition, the cleaning water 13 or ozone water 125 may contain a hydrogen peroxide solution as well as the cleaning agent and hot water 9. By adding the hydrogen peroxide solution, the activity for oxidation-decomposing organic substances, inorganic substances and the like that the hydrogen peroxide solution has increases the activity for oxidation-decomposing organic substances, inorganic substances and the like that ozone has, and enhances bactericidal activity. Alternately, as well as the cleaning agent and hot water 9, the cleaning water 13 or ozone water 125 may contain titanium oxide alone having the photocatalyst function, apatite having the adsorption function, photocatalyst-apatite having both the photocatalyst function and the adsorption function, or a photocatalyst function material comprised of a complex thereof or the like to enhance cleaning and bactericidal activity. Further, to cause the photocatalyst function material to exert the function effectively, an ultraviolet lamp may be disposed inside the cleaning and sterilizing container 14 to apply ultraviolet rays.

The above-mentioned Embodiment shows the example of using the cleaning sterilization apparatus of the invention mainly in cleaning sterilization of used medical instruments 16, and other than the medical instruments, it is possible to apply to cleaning sterilization of various articles. For example, it is possible to apply to barber/hair salon instruments, hygiene items, care items, cooking devices, etc.

### Industrial Applicability

The cleaning sterilization apparatus according to the invention increases the duration of ozone bubbles contained in ozone-mixed water, sterilizes an object to be cleaned such as a medical instrument with decomposition/oxidation action enhanced, and further, is also capable of being used in cleaning and sterilization of articles for which hazardous chemicals cannot be used.

### Description of Symbols

- 1:: Cleaning sterilization apparatus
- 2:: Apparatus housing
- 3:: Cleaning and sterilizing unit
- 4:: Operating panel
- 5:: Cleaning agent supply unit
- 6:: Hot water/cold water supply unit
- 7:: Dry unit
- 8:: Cold water
- 9:: Hot water
- 10:: Squirt/drain unit
- 11:: Control unit
- 12:: Cleaning and sterilizing start button
- 13:: Cleaning water
- 14:: Cleaning and sterilizing container
- 15:: Water level sensor
- 16:: Medical instrument
- 17:: Basket
- 18:: Cover
- 19:: Panel body
- 20:: Setting button
- 21:: Display
- 22:: Buzzer
- 23:: Cover
- 24:: Cleaning agent container
- 25:: Pipe
- 26:: Cleaning agent supply pump
- 27:: Cover
- 28:: Hot water container
- 29:: Thermostat
- 30:: Pipe
- 31:: Hot water supply pump
- 32:: Cold water container
- 33:: Pipe
- 34:: Cold water supply pump
- 35:: Intake shutter
- 36:: Intake duct
- 37:: Intake fan mechanism
- 38:: Heater
- 39:: Exhaust shutter
- 40:: Exhaust duct
- 41:: Rotating nozzle
- 42:: Pipe
- 43:: Circulating pump
- 44:: Drain pipe
- 45:: Drain pump
- 101:: Ozone water generating apparatus
- 104:: Treatment-target water
- 114:: Stirring portion
- 117:: Incurrent plate
- 118:: Protrusion
- 121:: Stirring plate
- 123:: Excurrent plate
- 124:: Protrusion
- 125:: Ozone water
- 130:: Stirring surface
- 131:: Stirring block
- 132:: Cylindrical portion
- 133:: Taper portion
- 134:: Hole

## Claims

1. A cleaning sterilization apparatus which cleans an object to be cleaned by squirting cleaning water with the object to be cleaned held inside a container, while sterilizing and disinfecting the object to be cleaned by oxidation-decomposing organic substances such as a virus, bacteria or the like adhering to the object to be cleaned using ozone water, comprising:
cleaning water supply means for supplying cleaning water; ozone water generating means for generating ozone water to supply; and
squirting means for squirting the cleaning water and the ozone water toward the object to be cleaned inside the container,
wherein the ozone water generating means is provided with a mixing pump which takes in ozone and water to mix, and generates ozone-mixed water with ozone mixed into water,
ozone supply means for supplying ozone to the mixing pump,
stirring means for colliding the ozone-mixed water supplied from the mixing pump sequentially with a plurality of protrusions with running-water pressure applied inside an enclosed running-water channel with the plurality of protrusions arranged therein, and thereby making ozone contained in the ozone-mixed water finer to generate ozone water, and
a line mixer which causes the stirring means to circulate and stir the ozone-mixed water generated by the mixing pump and thereby adjusts particle diameters of ozone contained in the ozone-mixed water.

2. The cleaning sterilization apparatus according to claim 1, wherein the cleaning sterilization apparatus squirts the ozone water to the object to be cleaned by the squirting means while circulating the ozone water, and thereby sterilizes and disinfects the object to be cleaned inside the container.

3. The cleaning sterilization apparatus according to claim 1, wherein the cleaning sterilization apparatus circulates the ozone water at a predetermined flow rate with the object to be cleaned immersed in the ozone water, and thereby sterilizes and disinfects the object to be cleaned inside the container.

4. The cleaning sterilization apparatus according to claim 3, further comprising:
ultrasonic vibration means for vibrating the ozone water at a frequency of an ultrasonic band inside the container.

5. The cleaning sterilization apparatus according to claim 1, wherein the stirring means has a circular stirring plate with a plurality of protrusions spaced circumferentially formed therein, collides the ozone-mixed water sequentially with the plurality of protrusions, and thereby makes ozone contained in the ozone-mixed water finer.

6. The cleaning sterilization apparatus according to claim 1, wherein the stirring means has a stirring block in the shape of a cylinder with a plurality of protrusions spaced circumferentially formed in a tapered inner surface of the cylinder, collides the ozone-mixed water sequentially with the plurality of protrusions, and thereby makes ozone contained in the ozone water finer.

7. The cleaning sterilization apparatus according to claim 3, wherein particle diameters of ozone bubbles inside the ozone water are controlled to within a desired size range by adjusting the running-water pressure inside the stirring means by the mixing pump and time of circulation stirring by the line mixer.

8. The cleaning sterilization apparatus according to claim 7, wherein a discharge pressure of the ozone-mixed water by the mixing pump which is supplied to the stirring means by the mixing pump ranges from 3 to 8 atmospheres.

9. The cleaning sterilization apparatus according to claim 1, wherein the desired size range of particle diameters of the ozone bubbles ranges from 4 to 50 µm.

10. The cleaning sterilization apparatus according to claim 1, wherein the mixing pump takes in the cleaning water and mixes the cleaning water with the ozone water, and the squirting means squirts the cleaning water and the ozone water toward the object to be cleaned.

11. The cleaning sterilization apparatus according to claim 1, wherein the cleaning water contains a hydrogen peroxide solution as well as the cleaning agent.

12. The cleaning sterilization apparatus according to claim 1, wherein as well as the cleaning agent, the cleaning water contains a photocatalyst, apatite or photocatalyst-apatite alone or a complex thereof.

13. The cleaning sterilization apparatus according to claim 1, wherein the ozone supply means generates an ozone gas with an ozone concentration ranging from 70 to 120 g/m3 to supply to the mixing pump.
